Europäisches Patentamt

European Patent Office

Office européen des brevets

(11) Publication number: **0 347 500 B1**

# EUROPEAN PATENT SPECIFICATION

(45) Date of publication of patent specification: **25.08.93**　(51) Int. Cl.5: **C07C 15/14**, C07C 6/12

(21) Application number: **88305770.5**

(22) Date of filing: **24.06.88**

The file contains technical information submitted after the application was filed and not included in this specification

(54) **Method of making ethylbiphenyls.**

(43) Date of publication of application:
**27.12.89 Bulletin 89/52**

(45) Publication of the grant of the patent:
**25.08.93 Bulletin 93/34**

(84) Designated Contracting States:
**BE DE FR GB NL**

(56) References cited:
**EP-A- 0 202 752**
**GB-A- 1 350 893**

**CHEMICAL ABSTRACTS, vol. 108, no. 13, March 1988, page 578, abstract no. 111947g, Columbus, Ohio, US; & JP-A-62 129 229**

(73) Proprietor: **NIPPON STEEL CHEMICAL CO., LTD.**
**13-16 Ginza 5-chome Chuo-ku**
**Tokyo(JP)**

(72) Inventor: **Sakura, Kathuhiko**
**9-2-21 Denenchoufu Minami**
**Ota-Ku Tokyo(JP)**
Inventor: **Takeuchi, Genki**
**4-5-301 Nakai-2-chome Kokurakita-Ku**
**Kitakyushu-shi Fukuoka(JP)**
Inventor: **Takeshita, Naoko**
**1010-4 Doujouji**
**Yukuhashi-shi Fukuoka(JP)**

(74) Representative: **Calamita, Roberto et al**
**Frank B. Dehn & Co., Imperial House, 15-19 Kingsway**
**London WC2B 6UZ (GB)**

## Description

This invention relates to a method of making ethylbiphenyls which are useful as heat transfer fluids.

Ethylbiphenyls are made by the ethylation of biphenyl; for example, Japanese Patent Publication No. 15,945-1972 (US Patent No. 3,636,179) discloses a method of making ethylbiphenyls by the catalytic transalkylation of biphenyl and ethylated benzenes containing ethylbenzenes, diethylbenzenes, triethylbenzenes, and tetraethylbenzenes in the presence of dissolved $AlCl_3$.

The Friedel-Crafts reaction using $AlCl_3$, however, generally possesses the following defects:

(1) The step for water washing and neutralization are required for the removal of $AlCl_3$ upon completion of the reaction, and acidic waste water is generated in large quantities;

(2) The reaction equipment must be made acid-resistant;

(3) The reaction is hard to carry out on a continuous basis; and

(4) The reuse of the spent $AlCl_3$ is difficult. The aforesaid method is no exception in this respect.

Other method in Japanese Kokai Document Nos. 156,222-1981 and 1,869-1972 (GB Patent No. 1,350,893) described the manufacture of ethylbiphenyls by the reaction of biphenyl with ethylene in the presence of a solid acid catalyst. They are, however, not readily applicable on an industrial scale because such solid acid catalyst has a short life and the methods are difficult to practice in a fixed-bed flow reactor.

The present inventors have conducted studies in search of remedies to the aforesaid defects, found that solid acid catalysts can be as reactive as $AlCl_3$ and yet free of the shortcomings of $AlCl_3$, and completed this invention.

An object of this invention is to provide a method of making ethylbiphenyls which as effective as the Friedel-Crafts reaction using the conventional $AlCl_3$ catalyst, generates no acidic waste water, requires no costly materials for the reaction equipment, and uses reusable catalysts of a long life.

Another object of this invention is to provide a method of making ethylbiphenyls which can be practiced on a continuous basis in a fixed-bed flow reactor.

Still another object of this invention is to provide a method of making ethylbiphenyls which prolongs the life of a solid acid catalyst to an industrially viable extent by the transethylation of biphenyl or by a combination of the ethylation and transethylation of biphenyl in the presence of such solid acid catalyst.

This invention thus relates to a method of making ethylbiphenyls which applies a solid acid as catalyst to the ethylation of biphenyl with polyethylbenzenes or with ethylene and polyethylbenzenes.

The solid acid catalysts useful for this invention are silica-alumina, preferably a silica-alumina mixture having a mole ratio silica/alumina of 2000 to 0.2, and Y zeolite, known as exceedingly strong solid acids.

The catalysts should preferably have from 0.1 mole to 3 moles of acid sites per Kg, each mole of the acid sites showing heat of adsorption of ammonia of 85 kilojoules or more. Industrially sufficient conversion is difficult to obtain with acid sites of 0.1 mole per Kg or less while carbonaceous deposits from faster and the catalyst reduce its activity faster with acid sites of 3 moles per Kg or more.

The reaction temperature is set in the range of from 130 to 380°C, preferably from 150 to 350°C, and more preferably from 200 to 320°C. Secondary reactions begin to accompany the ethylation above 380°C while the convertion is not sufficiently high below 130°C.

The reaction pressure is from ambient to 30 kg/cm$^2$ • G, preferably from 2 to 20 kg/cm$^2$ • G. A pressure above 30kg/cm$^2$ • G is allowable, but is not necessary.

The optimal reaction time or contact time varies with the reaction temperature chosen. A batch reaction should preferably run from 1 to 3 hours, and a continuous reaction should preferably run a weight hourly space velocity of from 0.5 to 3 kg/kg • hr. An unnecessarily long reaction time will cause the undesirable deethylation of the reaction products.

The quantity of feed polyethylbenzenes in the production of monoethylbiphenyls is chosen so that from 0.3 to 4 moles, preferably from 0.5 to 2 moles of the ethyl groups or (ethyl group + ethylene) is present per each mole of (biphenyl ring + benzene ring). More biphenyl would remain unchanged if this mole ratio fell below 0.3 while less monoethylbiphenyls and more polyethylbiphenyls would form if the ratio exceeded 4. In the cases where the intended product is diethylbiphenyls or higher polyethyldiphenyls, from 1 to 4 moles, preferably from 1.5 to 3 moles of the ethyl group of (ethyl group + ethylene) should be present per each mole of (biphenyl ring + benzene ring). Moreover, when polyethylbiphenyls produced are returned to a reaction system, the ethyl groups and biphenyls thereof are taken into account as well.

It is desirable for the feed polyethylbenzenes to contain from 1.5 to 4 moles, preferably from 2 to 3 moles, of the ethyl group per mole of benzene ring. When a mixed feed of polyethylbenzenes and ethylene is used, it is desirable that 2 moles or less, preferably from 0.2 to 1 mole, of ethylene is present for each mole of the ethyl group in the polyethylbenzenes. A larger quantity of ethylene forms more carbonaceous deposits on the catalyst and shortens the catalyst life.

The method of this invention can be practiced in a flow or batch mode. A fixed-bed flow reactor is suitable for large-scale production and a batch reactor for small-scale one. In either case, the catalyst can be separated readily from the reaction products. The reaction equipment may be constructed of stainless steel equivalent to SUS 304 and requires no acid-resistant glass lining.

When the reaction is carried out batch-wise, the spent catalyst separated after the reaction is reused with or without regeneration in the usual manner by firing at 500 °C or so in a stream of air diluted with nitrogen.

On the other hand, in a continuous operation using a fixed-bed flow reactor, plural reactors are provided in parallel between the feed line and the product line; as soon as the catalyst in one of the reactors is deactivated, the process flow is switched to another reactor containing fresh catalyst, the spent catalyst is regenerated or replaced and used again when the fresh catalyst in turn loses its activity. The product line is connected to a distillation column, preferably several in series, and the products withdrawn from the reactor are continuously separated and purified into the product ethylbiphenyls. The biphenl and polyethylbenzenes recovered unchanged and polyethylbiphenyls produced are returned to the reactors as recycle feed.

The method of this invention for making ethylbiphenyls with the use of solid acid catalysts differs markedly from the conventional method using the $AlCl_3$ catalyst and offers advantages of industrial significance in that it generates no acidic waste water, requires no costly construction material for the equipment, and permits reuse of the catalyst.

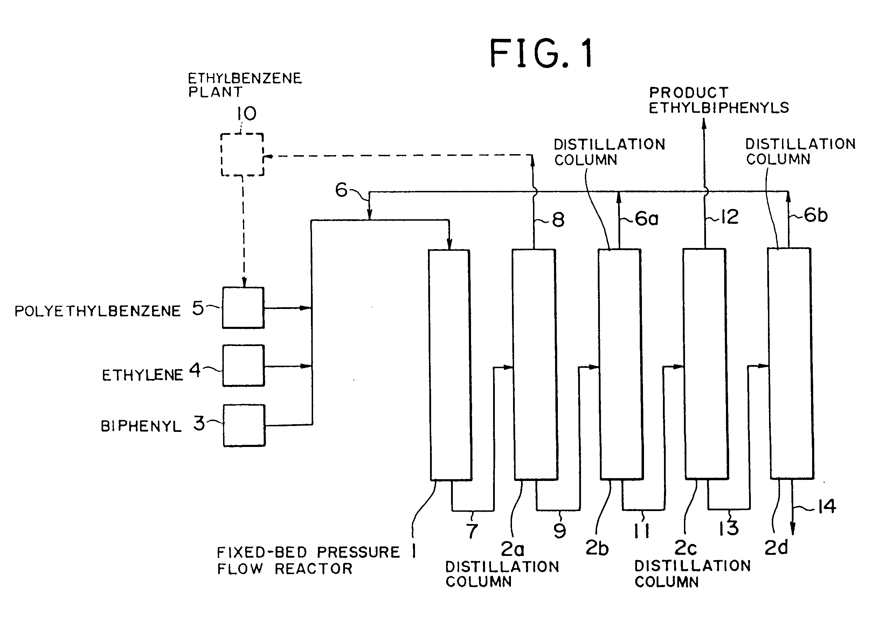

Figure 1 is the flowsheet for Example 7 in which ethylbiphenyls are manufactured continuously in a flow reactor embodying the method of this invention.

This invention is explained with reference to the accompanying examples.

Examples 1 ~ 4

Biphenyl (BP), polyethylbenzenes (PEB), and a silica-alumina catalyst (0.46 mole per Kg of acid sites with heat of adsorption of ammonia of 8.5 kilojoules per mole or more) were introduced into a pressure reactor equipped with a stirred and allowed to react under the conditions shown in Table 1. After completion of the reaction, the reaction mixture was filtered to separate the silica-alumina catalyst from the reaction products. The composition by weight of the compounds containing biphenyl ring in the reaction products is shown in Table 2.

The polyethylbenzenes used as feed were by-products in the manufacture of ethylbenzene.

Table 1

| | BP (Parts by weight) | PEB (Parts by weight) | Catalyst (Parts by weight) | Et Group/ (BPR + BR) Mole Ratio | Reaction Temperature (°C) | Reaction Time (h) | Reaction Pressure (kg/cm²·G) |
|---|---|---|---|---|---|---|---|
| Example 1 | 92.4 | 160.9 | 40 | 1.07 | 250 | 2 | 3 |
| " 2 | 107.8 | 117.3 | 40 | 0.89 | 300 | 2 | 10 |
| " 3 | 92.4 | 160.9 | 40 | 1.07 | 300 | 2 | 10 |
| " 4 | 38.5 | 134.0 | 40 | 1.28 | 300 | 2 | 10 |

BP: Biphenyl, PEB: Polyethylbenzenes, BPR: Biphenyl ring, BR: Benzene ring.

Table 2

| | BP (wt%) | EBP (wt%) | | | DiEBP (wt%) | TrEBP (wt%) | TeEBP (wt%) | 9-HF (wt%) | Othersrts (wt%) | Total (wt%) |
|---|---|---|---|---|---|---|---|---|---|---|
| | | meta | para | Subtotal | | | | | | |
| Example 1 | 35.6 | 14.8 | 29.2 | 44.0 | 12.2 | 4.1 | 0.8 | 1.0 | 2.2 | 100 |
| " 2 | 31.0 | 26.4 | 16.4 | 42.8 | 10.9 | 6.4 | 1.1 | 7.1 | 0.7 | 100 |
| " 3 | 18.5 | 22.0 | 14.1 | 36.1 | 19.7 | 11.9 | 6.6 | 5.9 | 1.2 | 100 |
| " 4 | 12.4 | 18.1 | 13.2 | 31.3 | 23.4 | 15.5 | 11.1 | 4.0 | 2.3 | 100 |

EBP: Ethylbiphenyls, DiEBP: Diethylbiphenyls, TrEBP: Triethylbiphenyls, TeEBP: Tetraethylbiphenyls, 9-HF: 9-Methylfluorene.

Example 5

A fixed-bed pressure flow reactor was filled with Y zeolite (acid sites, 1.19 mole per Kg) and a mixture of biphenyl and diethylbenzenes at a mole ratio of 1:4 was fed at 250 °C and a liquid hourly space velocity of 1ℓ/ℓ. hr. The effluents from the reactor accumulated over a period from the 24th to 32nd hour and those from 64th to 72 nd hour after the start of the reaction were analyzed and the results are shown in Table 3.

Table 3 (Example 5)

| Sampling Time (Hour from start) | BP (wt%) | EBP (wt%) | | | | DiEBP (wt%) | TrEBP (wt%) | TeEBP (wt%) | 9-MF (wt%) | Others (wt%) | Total (wt%) |
|---|---|---|---|---|---|---|---|---|---|---|---|
| | | ortho | meta | para | Subtotal | | | | | | |
| 24~32h | 12.3 | 0.8 | 18.3 | 15.8 | 34.9 | 35.7 | 11.7 | 1.2 | 3.0 | 1.2 | 100 |
| 64~72h | 12.6 | 0.8 | 18.1 | 17.5 | 36.4 | 33.9 | 11.4 | 1.1 | 3.8 | 0.8 | 100 |

Example 6

Into a pressure reactor equipped with a stirrer were introduced 55 g (0.38 mole) of biphenyl (BP) , 95g (0.71 mole) of a mixture of diethylbenzene isomers (DiEB), and 45 g of the silica-alumina catalyst, same as the one used in Example 1, and the transethylation reaction was carried out at an ethyl groups to aromatic groups mole ratio of 1.33 and at a catalyst to oil weight ratio of 0.30 by heating the mixture to 350°C over a period of 105 minutes and maintaining the temperature at this level thereafter. Sampling was made periodically to analyze the reaction mixture. The results are shown in Table 4.

## Table 4 (Example 6)

| Reaction Time After Temperature Rise (min.) | 0 | 60 | 120 | 180 | 240 |
|---|---|---|---|---|---|
| BP   Conversion (wt%) | 43.3 | 78.0 | 81.5 | 82.0 | 82.3 |
| Ratio of Monoethyl Isomers (%) | | | | | |
| o-EBP | 5.79 | 5.76 | 5.55 | 5.58 | 5.37 |
| m-EBP | 58.80 | 61.42 | 61.53 | 61.15 | 61.21 |
| p-EBP | 35.41 | 32.82 | 32.92 | 33.27 | 33.42 |
| Ratio of Diethyl Isomers (%) | | | | | |
| ①-DiEBP | 0.76 | 0.81 | 0.94 | 0.43 | 1.38 |
| ②-DiEBP | | 0.19 | 0.35 | 0.31 | 0.80 |
| ③-DiEBP | 6.85 | 8.17 | 9.20 | 8.23 | 8.39 |
| ④-DiEBP | 5.63 | 6.14 | 5.58 | 5.59 | 5.16 |
| 3,5-DiEBP | 14.11 | 13.50 | 12.81 | 12.91 | 12.82 |
| 3,3'-DiEBP | 30.95 | 31.66 | 32.76 | 32.70 | 32.30 |
| 3,4'-DiEBP | 33.23 | 31.81 | 30.63 | 32.02 | 31.37 |
| 4,4'-DiEBP | 8.73 | 7.73 | 7.71 | 7.80 | 7.78 |

Example 7

As illustrated in Fig. 1, fixed-bed pressure flow reactor 1 was filled with the silica-alumina catalyst, same as the one used in Example 1, and the product line thereafter was connected to distillation columns 2a, 2b, 2c, and 2d in series. The feeds, biphenyl 3, ethylene 4, and polyethylbenzene 5, were charged to reactor 1 together with recycle stream 6 which is a mixture of recycle stream 6a from the top of distillation column 2b and recycle stream 6b from the top of distillation column 2d. The reaction was carried out at 300°C and a weight hourly space velocity of 2 Kg/Kg • hr. The mole ratio of (ethyl groups + ethylene) to (biphenyl rings + benzene rings) was 1.16 in reactor 1.

Reaction mixture 7 flowing through the reactor and emerging from the bottom thereof was charged to distillation column 2a, and top fraction 8 and bottom fraction 9 were taken out from the top and the bottom of distillation column 2a respectively. Top fraction 8 was sent to ethylbenzene plant 10 where it is a supply source of polyethylbenzene 5.

Bottom fraction 9 was charged to distillation column 2b and recycle stream 6a mainly containing biphenyls and diethylbenzenes was withdrawn from the top while fraction 11 was withdrawn from the bottom.

Bottom fraction 11 was charged to distillation column 2c where ethylbiphenyl 12 mainly consisting of ethylbiphenyls (EBP) and diethylbiphenyls (DiEBP) was withdrawn from the top and fraction 13 mainly consisting of triethylbiphenyls (TrEBP) and tetraethylbiphenyls (TeEBP) was withdrawn from the bottom.

Fraction 13 was then charged to distillation column 2d and recycle stream 6b was taken out from the top while residue 14 was taken out from the bottom.

When triethylbiphenyl is needed as product, recycle stream 6b is withdrawn from the side of distillation column 2d and the triethylbiphenyl is taken out from the top.

The material balance in the flowsheet of figure 1 is shown in Table 5.

Table 5 (Example 7)

| Compound | Sample No. (Corresponding to No. In fig. 1) | | | | | | | | | | | | |
|---|---|---|---|---|---|---|---|---|---|---|---|---|---|
| | 3 | 4 | 5 | 6 | 7 | 8 | 9 | 6a | 11 | 12 | 13 | 6b | 14 |
| E | | 38 | | | | | | | | | | | |
| B | | | | | 118 | 118 | | | | | | | |
| EB | | | 6 | | 268 | 268 | | | | | | | |
| DiEB | | | 507 | 122 | 126 | 4 | 122 | 122 | | | | | |
| TrEB | | | 39 | 16 | 16 | | 16 | 16 | | | | | |
| TeEB | | | | 5 | 5 | | 5 | 5 | | | | | |
| Subtotal of Benzen Ring Compounds | 817 | | 552 | 143 | 533 | 390 | 143 | 143 | | | | | |
| BP | | | | 290 | 294 | | 294 | 290 | 4 | 4 | | | |
| EBP | | | | 3 | 573 | | 573 | 3 | 570 | 570 | | | |
| 9-HF | | | | | 60 | | 60 | | 60 | 60 | | | |
| DiEBP | | | | 11 | 313 | | 313 | | 313 | 302 | 11 | 11 | |
| TrEBP | | | | 180 | 189 | | 189 | | 189 | 9 | 180 | 180 | |
| TeEBP | | | | 83 | 105 | | 105 | | 105 | | 105 | 83 | 22 |
| Others | | | | 5 | 53 | | 53 | | 53 | 45 | 8 | 5 | 3 |
| Subtotal of Biphenyl Ring Compounds | 817 | | | 572 | 1587 | | 1587 | 293 | 1294 | 990 | 304 | 279 | 25 |
| Total | 817 | 38 | 552 | 715 | 2120 | 390 | 1730 | 436 | 1294 | 990 | 304 | 279 | 25 |

8

## Claims

1. A method of making ethylbiphenyls which comprises reacting biphenyl with polyethylbenzenes or with ethylene and polyethylbenzenes in the presence of a solid acid catalyst, said solid acid catalyst being at least one member selected from the group consisting of silica-alumina and Y zeolite.

2. A method as claimed in claim 1, wherein biphenyl is reacted with ethylene and polyethylbenzenes, with 2 moles or less of ethylene present for each mole of ethyl group in said polyethylbenzenes.

3. A method as claimed in claim 1 or claim 2, wherein the reaction temperature is from 130 to 380 °C.

4. A method as claimed in any one of the preceding claims, wherein the polyethylbenzenes are used in amounts such that there are 0.3 to 4 moles of ethyl groups per each mole of (biphenyl ring + benzene ring).

5. A method as claimed in any one of claims 1 to 4, wherein the polyethylbenzenes and ethylene are present in amounts such that there are 0.3 to 4 moles (ethyl groups + ethylene) per each mole of (biphenyl ring + benzene ring).

6. A method as claimed in any one of the preceding claims, wherein the solid acid catalyst possesses from 0.1 to 3.0 moles of acid sites, said acid site showing a heat of adsorption of ammonia of 85 kilojoules per mole or more.

7. A method as claimed in any one of the preceding claims, which comprises continuously charging biphenyl and polyethylbenzenes or biphenyl, ethylene, and polyethylbenzenes to a fixed-bed flow reactor packed with a solid acid catalyst, said solid acid catalyst being at least one member selected from the group consisting of silica-alumina and Y zeolite, continuously charging the reaction mixture withdrawn from said reactor to a distillation apparatus, recovering a fraction mainly containing ethylbiphenyls and diethylbiphenyls or additionally triethylbiphenyls as products, and charging a fraction mainly containing the recovered biphenyl and diethylbenzenes and a fraction mainly containing triethylbiphenyls and tetraethylbiphenyls or a fraction mainly containing tetraethylbiphenyls to said reactor as recycle raw materials.

8. A method as claimed in claim 7, wherein said distillation apparatus consists of plural distillation columns connected in series.

## Patentansprüche

1. Verfahren zur Herstellung von Ethylbiphenylen, das die Umsetzung von Biphenyl mit Polyethylbenzolen oder mit Ethylen und Polyethylbenzolen in Gegenwart eines festen sauren Katalysators umfaßt, wobei der feste saure Katalysator zumindest einer aus der Gruppe, bestehend aus Siliciumdioxid-Aluminiumoxid und Y-Zeolith, ist.

2. Verfahren gemäß Anspruch 1, worin Biphenyl mit Ethylen und Polyethylbenzolen umgesetzt wird, wobei für jedes Mol Ethylgruppe in den Polyethylbenzolen zwei Mole oder weniger an Ethylen vorliegen.

3. Verfahren gemäß Anspruch 1 oder 2, worin die Reaktionstemperatur 130 bis 380 °C beträgt.

4. Verfahren gemäß einem der vorhergehenden Ansprüche, worin die Polyethylbenzole in derartigen Mengen verwendet werden, daß 0,3 bis 4 Mol Ethylgruppen je Mol (Biphenylring + Benzolring) vorliegen.

5. Verfahren gemäß einem der Ansprüche 1 bis 4, worin die Polyethylbenzole und Ethylen in derartigen Mengen vorliegen, daß 0,3 bis 4 Mole (Ethylgruppen + Ethylen) je Mol (Biphenylring + Benzolring) vorliegen.

**6.** Verfahren gemäß einem der vorhergehenden Ansprüche, worin der feste saure Katalysator 0,1 bis 3,0 Mol saure Stellen besitzt, wobei diese sauren Stellen eine Adsorptionswärme von Ammoniak von 85 Kilojoules je Mol oder mehr aufweisen.

**7.** Verfahren gemäß einem der vorhergehenden Ansprüche, das die kontinuierliche Zufuhr von Biphenyl und Polyethylbenzolen oder Biphenyl, Ethylen und Polyethylbenzolen zu einem Festbettströmungsreaktor, der mit einem festen sauren Katalysator bepackt ist, wobei der feste saure Katalysator zumindest einer aus der Gruppe, bestehend aus Siliciumdioxid-Aluminiumoxid und Y-Zeolith ist, die kontinuierliche Zufuhr der aus dem Reaktor abgezogenen Reaktionsmischung zu einer Destillationsapparatur, die Gewinnung einer hauptsächlich Ethylbiphenyle und Diethylbiphenyle oder zusätzlich Triethylbiphenyle als Produkte enthaltenden Fraktion und die Zufuhr einer hauptsächlich das gewonnene Biphenyl und die gewonnenen Diethylbenzole enthaltenden Fraktion und einer hauptsächlich Triethylbiphenyle und Tetraethylbiphenyle enthaltenden Fraktion oder einer hauptsächlich Tetraethylbiphenyle enthaltenden Fraktion zu dem Reaktor als Recyclisierungsrohmaterialien umfaßt.

**8.** Verfahren gemäß Anspruch 7, worin die Destillationsapparatur aus mehreren in Reihe verbundenen Destillationskolonnen besteht.


**Revendications**

**1.** Procédé de préparation d'éthylbiphényles, qui comprend la réaction du biphényle avec des polyéthylbenzènes ou avec l'éthylène et des polyéthylbenzènes en présence d'un catalyseur acide solide, ledit catalyseur acide solide étant au moins un élément choisi parmi les silice-alumines et la zéolite Y.

**2.** Procédé selon la revendication 1, dans lequel le biphényle est mis à réagir avec l'éthylène et les polyéthylbenzènes, à raison de 2 moles ou moins d'éthylène pour chaque mole de groupe éthyle dans lesdits polyéthylbenzènes.

**3.** Procédé selon la revendication 1 ou la revendication 2, dans lequel la température de réaction va de 130 à 380° C.

**4.** Procédé selon l'une quelconque des revendications précédentes, dans lequel les polyéthylbenzènes sont utilisés en quantités telles qu'il y ait 0,3 à 4 moles de groupes éthyle pour chaque mole de (cycle biphényle + cycle benzène).

**5.** Procédé selon l'une quelconque des revendications 1 à 4, dans lequel les polyéthylbenzènes et l'éthylène sont présents en quantités telles qu'il y ait 0,3 à 4 moles de (groupes éthyle + éthylène) pour chaque mole de (cycle biphényle + cycle benzène).

**6.** Procédé selon l'une quelconque des revendications précédentes, dans lequel le catalyseur acide solide possède de 0,1 à 3,0 moles de sites acides, lesdits sites acides présentant une quantité de chaleur d'absorption de l'ammoniaque de 85 kilojoules par mole ou plus.

**7.** Procédé selon l'une quelconque des revendications précédentes, qui comprend le chargement en continu du biphényle et des polyéthylbenzènes ou du biphényle de l'éthylène et des polyéthylbenzènes dans un réacteur à écoulement à lit fixe, garni avec un catalyseur acide solide, ledit catalyseur acide solide étant au moins un élément choisi parmi la silice-alumine et la zéolite Y, le chargement continu du mélange réactionnel retiré dudit réacteur dans un appareil de distillation, la récupération d'une fraction contenant principalement les éthylbiphényles et les diéthylbiphényles ou en plus les triéthylbiphényles en tant que produits, et le chargement d'une fraction contenant majoritairement le biphényle et les diéthylbenzènes récupérés et une fraction contenant principalement les triéthylbiphényles et les tétraéthylbiphényles ou une fraction contenant principalement les tétraéthylbiphényles dans ledit réacteur en tant que matériaux bruts de recyclage.

**8.** Procédé selon la revendication 7, dans lequel ledit appareil de distillation consiste en colonnes de distillation multiples reliées en séries.

# FIG.1

ETHYLBENZENE PLANT — 10

PRODUCT ETHYLBIPHENYLS

DISTILLATION COLUMN

DISTILLATION COLUMN

6

~8    ~6a    ~12    ~6b

POLYETHYLBENZENE 5

ETHYLENE 4

BIPHENYL 3

FIXED-BED PRESSURE 1
FLOW REACTOR

7    2a    9    2b    11    2c    13    2d    ~14

DISTILLATION COLUMN

DISTILLATION COLUMN

EP 0 347 500 B1